(19) Europäisches Patentamt
European Patent Office
Office européen des brevets

(11) **EP 4 635 403 A1**

(12) **EUROPEAN PATENT APPLICATION**

(43) Date of publication:
**22.10.2025 Bulletin 2025/43**

(21) Application number: **24170984.9**

(22) Date of filing: **18.04.2024**

(51) International Patent Classification (IPC):
***A61B 5/00*** *(2006.01)*

(52) Cooperative Patent Classification (CPC):
**A61B 5/4812; A61B 5/4809; A61B 5/7264;
G16H 50/20;** A61M 16/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **VAN DER AAR, Jaap Floris
Eindhoven (NL)**
• **VAN DEN ENDE, Daan Anton
Eindhoven (NL)**

• **DOS SANTOS DA FONSECA, Pedro Miguel
Ferreira
5656AG Eindhoven (NL)**
• **VAN GORP, Hans
Eindhoven (NL)**
• **PERI, Elisabetta
Eindhoven (NL)**
• **OVEREEM, Sebastiaan
Eindhoven (NL)**
• **VAN GILST, Merel Marietje
Eindhoven (NL)**

(74) Representative: **Philips Intellectual Property &
Standards
High Tech Campus 52
5656 AG Eindhoven (NL)**

(54) **COMPUTER-IMPLEMENTED METHOD, COMPUTER PROGRAM PRODUCT, AND AROUSAL DETECTION SYSTEM**

(57) There is provided a computer-implemented method for detecting an arousal in a sleep session of a subject. The computer-implemented comprises receiving a first signal comprising information about a first probability distribution of sleep stage classes during the first epoch. The computer-implemented comprises receiving a second signal comprising information about a second probability distribution of the sleep stage classes during the second epoch. The computer-implemented comprises determining a continuity metric representative of a difference between the first probability distribution and the second probability distribution. The computer-implemented comprises determining a presence of an arousal based on the continuity metric. Further, there is provided an arousal detection system for detecting an arousal in a sleep session of a subject. The arousal detection system comprises a processing system and a sensor interface. The processing system is configured to perform the computer-implemented method.

FIG. 3

EP 4 635 403 A1

**Description**

FIELD OF THE INVENTION

The invention relates to a computer-implemented method for detecting an arousal in a sleep session of a subject. Further, the invention relates to a computer program product and a computer-readable storage medium comprising the computer program product. Further, the invention relates to an arousal detection system for detecting an arousal in a sleep session of a subject. Further, the invention relates to a respiratory support device for providing a pressurized airflow to a subject, comprising the arousal detection system.

BACKGROUND OF THE INVENTION

Sleep-disordered breathing (SDB) conditions, such as OSA and CSA, are becoming increasingly common, and are particularly prevalent in older people, people with a high body mass index, smokers, heavy drinkers and people with conditions such as coronary artery disease, hypertension, and diabetes mellitus.

SDB conditions are often treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

Although PAP therapy helps to reduce sleep-disordered breathing, the PAP therapy may increase the number of arousals during sleep. Although arousals are natural and common during sleep, excessive amounts of arousals can cause sleep fragmentation. Sleep fragmentation can not only cause short term problems, such as daytime sleepiness, but is also associated with long-term problems, including diabetes, cardiovascular disease, and depression. Arousals can be observed in the brain and measured using electroencephalogram, EEG. Albeit more challenging, arousals can also be detected utilizing other physiological processes, such as respiratory signals, heart rate (variability), and muscle activity.

SUMMARY OF THE INVENTION

Although using EEG to measure arousals provides the most accurate results in view of the gold standard, EEG is obtrusive to the subject, because of the electrodes that are attached to the scalp of the subject during the EEG measurement. Some measurements of other physiological processes to observe arousals are less obtrusive, but provide a less accurate result than the EEG measurement.

It is an objective of the invention to determine the presence of arousals in an improved way.

According to a first aspect, there is provided a computer-implemented method for detecting an arousal in a sleep session of a subject. The computer-implemented comprises receiving a first signal comprising information about a first probability distribution of sleep stage classes during the first epoch. The computer-implemented comprises receiving a second signal comprising information about a second probability distribution of the sleep stage classes during the second epoch. The computer-implemented comprises determining a continuity metric representative of a difference between the first probability distribution and the second probability distribution. The computer-implemented comprises determining a presence of an arousal based on the continuity metric.

When determining the sleep stage of an epoch, a probability distribution is used that includes the probabilities of all the sleep stage classes for that epoch. The sleep stage class that is used to classify that epoch is the sleep stage class with the highest probability. The invention makes use of the insight that the probability distribution changes smoothly over multiple epochs during undisturbed sleep. However, in case an arousal occurs, there is an abrupt change in the probability distribution. This change becomes visible when looking at the difference between the first probability distribution of first epoch and the second probability distribution of the second epoch. A continuity metric is defined, which is representative of this difference. In case the continuity metric indicates the continuity between the first probability distribution and the second probability distribution is large, the difference between the first probability distribution and the second probability distribution is small. The small difference indicates undisturbed sleep between the first epoch and the second epoch. In case the continuity metric indicates the continuity between the first probability distribution and the second probability distribution is small, the difference between the first probability distribution and the second probability distribution is large. The large difference indicates a disruption of sleep between the first epoch and the second epoch. Such disruption is indicative of an arousal. Since there are many unobtrusive ways to measure sleep stages to generate the probability distributions, the presence of arousals can be accurately detected without the need of EEG measurements. As a result, the invention provides an improved way of determining the presence of arousals.

An epoch is an interval of a defined length. The defined length is, for example, between 1 and 60 seconds. For example, epochs have a defined length of 2, or 5, or 6, or 10 seconds. It is well-known to use epochs with a length of 30 seconds when determining sleep stages.

For example, the sleep stages are classified into two sleep stage classes, i.e., a wake sleep stage class and a non-wake sleep stage class. During the wake sleep stage, the subject is awake. During the non-wake sleep stage, the subject is asleep. For example, the sleep stages are classified into three classes. The three classes are a wake sleep stage class, a REM sleep stage class, and a non-REM sleep stage class. During the REM sleep stage class, the subject is asleep and has Rapid Eye Movements, whereas during the non-REM sleep stage class, the subject is asleep without Rapid Eye Movements. For example, the sleep stages are classified into four classes. The four classes are a wake sleep stage class, a REM sleep stage class, a light sleep stage class, and a deep sleep stage class. For example, the sleep stages are classified into five classes. The five classes are a wake sleep stage class, a REM sleep stage class, two light sleep stage classes N1 and N2, and a deep sleep stage class N3.

The first signal comprises information about the first probability distribution of sleep stage classes during the first epoch. The first probability distribution has multiple probabilities, one for each sleep stage class. For example, the first probability distribution has a total value of 1. The probabilities of the sleep stage classes have a value that ranges from 0-1. A value close to 0 represents a low probability that the subject has a sleep stage in that sleep stage class during the epoch. A value close to 1 represents a high probability that the subject has a sleep stage in that sleep stage class during the epoch. The sum of the probabilities of the sleep stage classes adds up to the total value of 1.

For example, the first signal comprises information from a hypnodensity. A hypnodensity, also referred to as a hypnodensity graph, comprises for each epoch of a sleep session the probability distribution of the sleep stage classes. The probability distribution is based on physiological measurements on the subject. In case the physiological measurements are indicative of one of the sleep stage classes with a high degree of certainty, the hypnodensity shows a high probability for that sleep stage class, while showing a low probability for the other sleep stage classes. In case the measurements are not indicative of any of the sleep stage classes with a high degree of certainty, the hypnodensity shows low probabilities for all sleep stage classes. For example, the hypnodensity is generated by an automated sleep stage classification model. An automated sleep stage classification model uses input from physiological measurements to classify each epoch in a sleep session to a sleep stage class. For each epoch, the automated sleep stage classification model generates a probability distribution representing the probability of each sleep stage class. The automated sleep stage classification model classifies the epoch to the sleep stage class with the highest probability. The total of the probability distributions for all epochs forms the hypnodensity.

For example, the second signal comprises the same information as the first signal, but for the second epoch instead of the first epoch. For example, the second signal comprises information from a hypnodensity, for example the same hypnodensity as the first signal. For example, the second signal is based on the hypnodensity that is part of the hypnogram on which the first signal is based.

The continuity metric is representative of a difference between the first probability distribution and the second probability distribution. For example, the continuity metric is based on a distance metric, such as a Euclidean distance, or a Cosine distance, or a Manhattan distance. For example, the continuity metric is based on a statistical distance that is representative of a distance between the first probability distribution and the second probability distribution. For example, the continuity metric is based on a divergence between the first probability distribution and the second probability distribution. For example, the continuity metric is based on a comparison between the probabilities for each sleep stage class in the first probability distribution and the probabilities for each sleep stage class the second probability distribution.

The presence of the arousal is based on the continuity metric. For example, based on the continuity metric, the presence of the arousal is determined during the first epoch and/or during the second epoch, and/or between the first epoch and the second epoch. For example, the arousal is determined to be present in case the continuity metric exceeds a threshold representing a large difference between the first probability distribution and the second probability distribution. For example, the arousal is determined not to be present in case the continuity metric does not exceed the threshold representing a large difference between the first probability distribution and the second probability distribution. For example, the presence of the arousal is determined based on a comparison of the continuity metric with a reference.

In an embodiment, the first epoch and the second epoch are adjacent to each other.

According to this embodiment, the first epoch and the second epoch are adjacent to each other. For example, the first epoch is directly before the second epoch. For example, the second epoch is directly before the first epoch. Determining the presence of the arousal based on the two adjacent epochs gives an improved insight on whether the subject suffers from sleep fragmentation.

In an embodiment, the first probability distribution is representative of a classification of the first epoch as one of the sleep stage classes. The second probability distribution is representative of a classification of the second epoch as another one of the sleep stage classes.

According to this embodiment, the sleep stage in the first epoch is different from the sleep stage in the second epoch. It is especially of interest from a clinical perspective to understand what happens during a sleep stage transition. During a sleep stage transition, the subject is in one sleep stage class in the first epoch and in another sleep stage class in the second epoch. Sleep stage transitions are a natural phenomenon during healthy sleep. However, in case the sleep stage transition occurs simultaneously with an arousal, the subject may suffer from sleep fragmentation. Especially in case the subject has many sleep stage transitions occurring simultaneously with an arousal, the subject may suffer from sleep fragmentation. Based on the insight from the sleep stage transition and whether or not an arousal is present, a professional caretaker can take appropriate action to improve the sleep of the subject.

In an embodiment, the continuity is based on a distance metric between the first probability distribution and the second probability distribution.

In an embodiment, the continuity is based on a derivative of the distance metric.

In an embodiment, determining the presence of the arousal comprises performing a comparison between the continuity metric and a reference metric, and determining the presence of the arousal based on the comparison.

According to this embodiment, the presence of the arousal is determined by comparing the continuity metric with a reference metric. By comparing the continuity metric with the reference metric, the presence of the arousal can be detected with improved accuracy. For example, the reference metric is representative of the presence of an arousal. For example, the reference metric is representative of the absence of an arousal. For example, the reference metric is representative of an average value for the continuity metric of a group of subjects that have the same condition as the subject. For example, the reference metric is representative of an average value for the continuity metric of a group of subjects that does not have the same condition as the subject. For example, the condition is sleep disordered breathing (SDB), or obstructive sleep apnea (OSA), or periodic limb movement disorder (PLMD). For example, the presence of the arousal is determined by comparing the continuity metric with a plurality of different reference metrics.

In an embodiment, the reference metric comprises a first reference continuity metric corresponding to the presence of an arousal.

In an embodiment, the reference metric comprises a second reference continuity metric corresponding to an absence of an arousal.

In an embodiment, the probability distribution comprises multiple probabilities, one for each of the sleep stage classes. The continuity metric is based on a difference between probabilities of corresponding sleep stage classes in the first epoch and the second epoch.

According to this embodiment, the continuity metric is based on the difference between what the probabilities are in the first epoch and what the probabilities are in the second epoch, for the different sleep stage classes. For example, there are four sleep stage classes: wake, REM, light sleep, and deep sleep. In this example, the difference between the probability of the wake sleep stage class in the first epoch and the probability of the wake sleep stage class in the second epoch is determined. Also, the difference between the probability of the REM sleep stage class in the first epoch and the probability of the REM sleep stage class in the second epoch is determined. Also, the difference between the probability of the light sleep stage class in the first epoch and the probability of the light sleep stage class in the second epoch is determined. Also, the difference between the probability of the deep sleep stage class in the first epoch and the probability of the deep sleep stage class in the second epoch is determined. The continuity is based on these four differences. In another example, there are three sleep stage classes: wake REM, and non-REM. In this example, the continuity is based on three differences: the difference between the probability of the wake sleep stage class in the first epoch and the probability of the wake sleep stage class in the second epoch, the difference between the probability of the REM sleep stage class in the first epoch and the probability of the REM sleep stage class in the second epoch, and the difference between the probability of the non-REM sleep stage class in the first epoch and the probability of the non-REM sleep stage class in the second epoch. Similarly, the continuity metric is based on the difference between what the probabilities are in the first epoch and what the probabilities are in the second epoch, for the different sleep stage classes in case there are two sleep stage classes or five sleep stage classes.

In an embodiment, the computer-implemented method comprises receiving at least one additional signal comprising information about at least one additional probability distribution of at least one additional epoch. The first epoch, the second epoch and the at least one additional epoch are within a timeframe of an arousal. The computer-implemented method comprises determining the continuity metric based on a difference between the first probability distribution, the second probability distribution, and the at least one additional probability distribution.

It is common that arousals occur and disappear within about 5 minutes or within about 2 minutes or within about 1 minute. Therefore, the timeframe of an arousal is, for example, 1 minute or 2 minutes or 5 minutes. During this timeframe, the brain of the subject has increased activity that triggers the arousal. During this timeframe, there is an increase in sympathetic activity, such as an increase in heart rate and/or an increase in blood pressure and/or an increase in breathing rate. At the end of the timeframe, the sympathetic activity returns to the state as prior to the arousal. For example, heart rate, blood pressure and breathing rate are substantially the same as prior to the arousal. The time frame may cover more epochs than only the first epoch and the second epoch. In this embodiment, more epochs are included to cover more or all of the timeframe of the arousal. This way, the differences of the probability distributions along a larger portion of the timeframe of the arousal can be determined, resulting in a more accurate determination on whether an arousal is present.

In an embodiment, the computer-implemented method defines the continuity with the formula:

$$\text{Continuity} = 1 - \frac{1}{2}\sum_i |p(x_i(t)) - p(x_i(t+1))|$$

wherein $p(x_i(t))$ resembles a probability for each sleep stage class in the first epoch,

wherein $p(x_i(t+1))$ resembles a probability for each sleep stage class in the second epoch.

Based on this embodiment, the continuity is defined in a clear way that provides a good measure on which the presence of an arousal can be based.

In an embodiment, the computer-implemented method comprises receiving a first physiological signal representative of a physiological property of the subject during the first epoch. The computer-implemented method comprises receiving a second physiological signal representative of the physiological property of the subject during the second epoch. The physiological property comprises information about the sleep stages. The computer-implemented method comprises generating the first probability distribution based on the first physiological signal. The computer-implemented method comprises generating the second probability distribution based on the second physiological signal.

For example, the physiological property comprises brain activity of the subject. The physiological signal is, for example, generated based on neurological signals of the subject. For example, the neurological signals are obtained via polysomnography (PSG), via electroencephalography (EEG), via electrooculography (EOG), via electromyography (EMG) or any combination of these. A sensor adapted to generate a sensor signal based on the neurological signals is, for example, mounted on a wearable device for the head or face, such as a headband. The first probability distribution and the second probability distribution are based on the neurological signals via use of an automated sleep stage classification model or via manual annotation.

For example, the physiological property is based on a surrogate measure of sleep. For example, the surrogate measure of sleep is based on changes in autonomic nervous system activity of the subject. A change in the autonomic nervous system activity is, for example, detected based on cardiac signals obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject. The transmissive PPG sensor is, for example, arranged on the finger of the subject. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject. The seismocardiographic sensor comprises, for example, an accelerometer mounted on the chest of the subject. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g., instantaneous heart rate). The input is, for example, input as raw data to the machine learning model.

For example, the physiological property is based on respiratory activity. Respiratory activity of the subject is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax or the abdomen. For example, the sensor adapted to measure respiratory activity comprises a pressure sensor mounted on the bed or mattress. For example, the sensor adapted to measure respiratory activity comprises a Doppler radar positioned near the subject. For example, the sensor for measuring respiratory activity comprises an accelerometer or a gyroscope mounted on the thorax, the abdomen and/or sternum of the subject.

In an embodiment, the computer-implemented method comprises using an automated sleep stage classification model for generating the first probability distribution based on the first physiological signal, generating the second probability distribution based on the second physiological signal, classifying the first epoch as one of the sleep stage classes, and classifying the second epoch as one of the sleep stage classes.

According to this embodiment, the use of an automated sleep stage classification model has two benefits. Firstly, the sleep stage of each epoch during the sleep session is automatically classified by the automated sleep stage classification model to one of the sleep stage classes. There is no need for any manual interaction. Therefore, the information about the sleep stages during the sleep session becomes available without substantial effort and costs. Secondly, automated sleep stage classification models make use of probability distributions when determining a sleep stage for an epoch. Because the probability distributions are already available, this allows for an easy and cost-effective way to determine the continuity metric.

In an embodiment, the physiological property is related to at least one of a brain activity, a cardiac property, and a respiratory property of the subject during the sleep session.

According to this embodiment, a selection is made for a desired physiological property. By using an appropriate automated sleep stage classification model, the probability distributions are generated. In case the physiological property relates to brain activity, the probability distributions are generated in an accurate way. The gold standard to determine the presence of arousals is using EEG measurements to detect the presence of cortical arousals. These EEG measurements measure brain activity to detect the presence of cortical arousals. By using an automated sleep stage classification model for generating the probability distributions based on brain activity as the physiological property, the outcome of the automated sleep stage classification model is highly comparable with the gold standard. However, the EEG measurements needed to detect the brain activity require a setup by a technical expert. Also, because of the many wired electrodes that are attached to the scalp of the subject to perform the EEG measurements, the EEG measurements provide reduced comfort for the subject, and therefore affect the sleep of the subject. A known automatic sleep stage classification model that is based on brain activity information is Somnolyzer. In case the subject is undergoing PAP therapy, respiratory information is available via a respiratory support device providing a pressurized airflow to the subject. The respiratory information, such as air pressure or airflow during inhaling and exhaling, breathing rate, respiratory effort, and/or breathing depth, provides information about the respiratory property. The respiratory information is used by an automated sleep stage classification model to determine the probability distributions. This way, there is no need to obtain additional measurement information from the subject, other than what is already available via the respiratory support device. A known automatic sleep stage classification model that is based on respiratory information is CardioRespiratory Sleep Staging, CReSS. CReSS is able to perform automatic sleep stage classification based on respiratory information as the only input. In case the physiological property relates to a cardiac property, the subject has, for example, a wearable device configured to obtain information about the cardiac property. Such wearable devices are readily available and can be worn with only limited discomfort to the subject. For example, the cardiac property comprises heart rate, or heart rate variability, or interbeat intervals. A known automatic sleep stage classification model that is based on cardiac information is CardioRespiratory Sleep Staging, CReSS. CReSS is able to perform automatic sleep stage classification based on cardiac information as the only input. For example, the physiological property comprises both a cardiac property and a respiratory property. A known automatic sleep stage classification model that is based on both cardiac information and respiratory information is CardioRespiratory Sleep Staging, CReSS.

According to a second aspect of the invention, there is provided a computer program product, comprising instructions which, when executed by a processing system, cause the processing system to carry out the computer-implemented method of the first aspect of the invention.

According to a third aspect of the invention, there is provided a computer-readable storage medium comprising instructions which, when executed by a processing system, cause the processing system to carry out the computer-implemented method of the first aspect of the invention.

According to a fourth aspect of the invention, there is provided an arousal detection system for detecting an arousal in a sleep session of a subject. The arousal detection system comprises a processing system and a sensor interface. The processing system is configured to perform the computer-implemented method of the first aspect of the invention. The sensor interface is coupled to the processing system. The sensor interface is configured to be coupled to at least one sensor for generating the physiological signal representative of the physiological property. The sensor interface is configured to provide the first physiological signal and the second physiological signal to the processing system. The arousal detection system comprises an output generator configured to generate an output signal representative of the presence of the arousal in response to the processing system determining the presence of the arousal.

In an embodiment, the arousal detection system comprises the at least one sensor configured to generate the physiological signal. The sensor is coupled to the sensor interface.

According to a fifth aspect of the invention, there is provided a respiratory support device for providing a pressurized airflow to a subject. The respiratory support device comprises the arousal detection system according to the fourth aspect. The respiratory support device further comprises an airflow source adapted to generate the pressurized air, and a patient interface adapted to provide the pressurized airflow to the subject.

BRIEF DESCRIPTION OF THE DRAWINGS

Exemplary embodiments will now be described, by way of example only, with reference to the following Figures, in which:

FIG. 1 depicts a respiratory support device according to a first embodiment of the invention;
FIG. 2 depicts an arousal detection system according to the first embodiment;
FIG. 3 depicts a computer-implemented method according to the first embodiment;
FIG. 4 depicts a plurality of probability distributions according to the first embodiment;
FIG. 5 depicts a computer-implemented method according to a second embodiment;
FIG. 6 depicts the continuity metric generated by a brain-based automated sleep stage classification model;

FIG. 7 depicts the continuity metric generated by an HRV-based automated sleep stage classification model;
FIGs 8-10 depict a probability of an arousal based on the continuity metric.

DETAILED DESCRIPTION OF EMBODIMENTS

It should be understood that the detailed description and specific examples, while indicating exemplary embodiments of the devices, systems, and methods, are intended for purposes of illustration only and are not intended to limit the scope of the invention. These and other features, aspects, and advantages of the devices, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

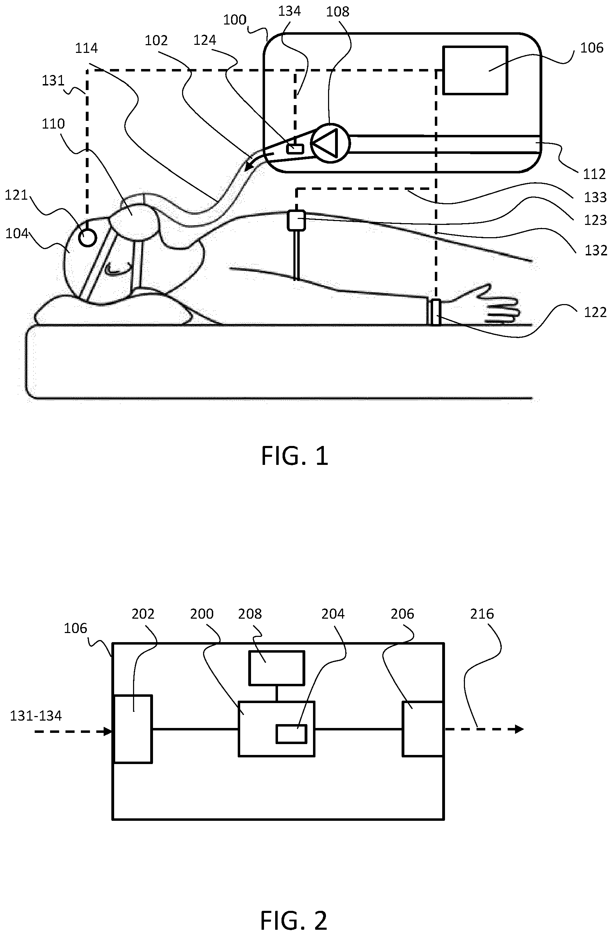

FIG. 1 depicts a respiratory support device 100 according to a first embodiment of the invention. The respiratory support device 100 is for providing a pressurized airflow 102 to a subject 104. The respiratory support device 100 comprises an arousal detection system 106, an airflow source 108 and a patient interface 110. The airflow source 108 is adapted to generate the pressurized airflow 102. The patient interface 110 is adapted to provide the pressurized airflow 102 to the subject 104.

The airflow source 108 draws air into the respiratory support device 100 via an air inlet 112. The airflow source 108 pressurizes the air from the air inlet 112 and provides the pressurized airflow 102 via an air delivery tube 114 to the patient interface 110. Via the patient interface 110, the pressurized airflow 102 is able to prevent or help to reduce a collapse of the upper airway of the subject 104.

FIG. 1 depicts four different sensors 121-124 to measure a physiological property of the subject 104 during a sleep session. Each of the four different sensors 121-124 is configured to generate a physiological signal representative of the physiological property. In an embodiment, only one of these sensors 121-124 is present. In another embodiment, a subset of two or three of these sensors 121-124 is present. A first sensor 121 is attached to the head of the subject 104. The first sensor 121 is adapted to generate a first sensor signal 131 representative of brain activity of the subject 104. The physiological property relates to the brain activity. For example, the first sensor 121 is an electroencephalographic (EEG) sensor. A second sensor 122 is arranged at the wrist of the subject 104. The second sensor 122 is adapted to generate a second sensor signal 132 representative of a cardiac property of the subject 104. For example, the cardiac property is a heart rate or a heart rate variability or an interbeat interval. The physiological property relates to the cardiac property. For example, the second sensor 122 is a photoplethysmographyic (PPG) sensor. A third sensor 123 is attached to the chest of the subject 104. The third sensor 132 is adapted to generate a third sensor signal 133 representative of a respiratory property of the subject 104. The physiological property relates to the respiratory property. For example, the respiratory property is a breathing rate, or a breathing depth. For example, the third sensor 123 comprises an accelerometer adapted to detect a movement of the chest of the subject 104. The movement of the chest is representative of a respiration effort of the subject 104. Respiration effort is an example of a respiratory property. A fourth sensor 124 is arranged in the path of the pressurized airflow 102. The fourth sensor 124 is adapted to generate a fourth sensor signal 134 based on the flowrate of the pressurized airflow 102. Due to inhalation and exhalation of the subject 104, the flowrate of the pressurized airflow 102 changes. As a result, the fourth sensor 124 is adapted to generate a fourth sensor signal 134 representative of a respiratory property of the subject 104. The physiological property relates to the respiratory property.

The first sensor signal 131, the second sensor signal 132, the third sensor signal 133, and the fourth sensor signal 134 or any subset of the first sensor signal 131, the second sensor signal 132, the third sensor signal 133, and the fourth sensor signal 134 are provided to the arousal detection system 106. The arousal detection system 106 is further explained in FIG. 2.

FIG. 2 depicts the arousal detection system 106 according to the first embodiment. The arousal detection system 106 is for detecting an arousal in a sleep session of the subject 104. The arousal detection system 106 comprises a processing system 200 and a sensor interface 202. The processing system 200 is configured to perform the computer-implemented method as is described further on. The sensor interface 202 is coupled to the processing system 200. The sensor interface 202 is configured to be coupled to at least one of the first sensor 121, the second sensor 122, the third sensor 123, and the fourth sensor 124. The first sensor 121, the second sensor 122, the third sensor 123, and/or the fourth sensor 124 are for generating the physiological signal representative of the physiological property. The sensor interface 202 is configured to provide the first physiological signal and the second physiological signal to the processing system 200. The arousal detection system 106 comprises an output generator 206 configured to generate an output signal 216 representative of the presence of the arousal in response to the processing system 200 determining the presence of the arousal.

For example, the processing system 200 comprises a memory 204. For example, the memory 204 is configured to store a computer program product. The computer program product comprises instructions which, when executed by the processing system 200, cause the processing system 200 to carry out the computer-implemented method.

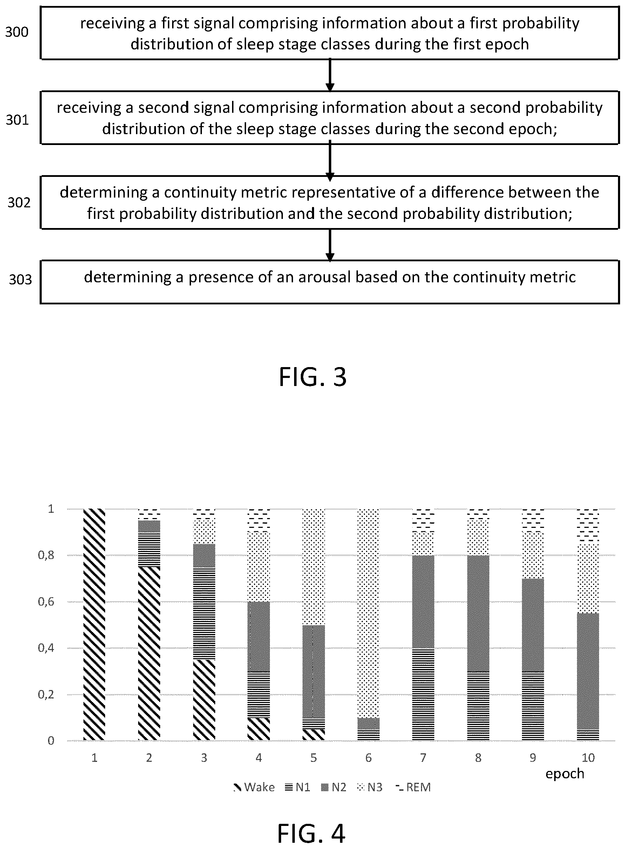

FIG. 3 depicts a computer-implemented method according to the first embodiment. The processing system 200 is configured to carry out the computer-implemented method. The computer-implemented method is for detecting an arousal in a sleep session of a subject 104. The computer-implemented method comprises, in 300, receiving a first signal comprising information about a first probability distribution of sleep stage classes during the first epoch. The computer-implemented method comprises, in 301, receiving a second signal comprising information about a second probability distribution of the sleep stage classes during the second epoch. The computer-implemented method comprises, in 302, determining a continuity metric representative of a difference between the first probability distribution and the second probability distribution. The computer-implemented method comprises, in 303, determining a presence of an arousal based on the continuity metric.

For example, the computer-implemented method comprises receiving a first physiological signal representative of a physiological property of the subject 104 during the first epoch. The computer-implemented method comprises receiving a second physiological signal representative of the physiological property of the subject 104 during the second epoch. The physiological property comprises information about a sleep stage of the subject 104. For example, the first physiological signal and the second physiological signal is one or more of the first sensor signal 131, the second sensor signal 132, the third sensor signal 133, and the fourth sensor signal 134. The computer-implemented method comprises generating the first probability distribution based on the first physiological signal. The computer-implemented method comprises generating the second probability distribution based on the second physiological signal. Optionally, the computer-implemented method comprises comprising using an automated sleep stage classification model 208, which is depicted in FIG. 2, for generating the first probability distribution based on the first physiological signal, for generating the second probability distribution based on the second physiological signal, for classifying the first epoch as one of the sleep stage classes; and for classifying the second epoch as one of the sleep stage classes.

The first physiological signal physiological is representative of a status or value of the physiological property of the subject 104 during the first epoch. The status or the value of the physiological property may be present only during the first epoch, or may extend beyond the first epoch to one or more adjacent epochs. The second physiological signal physiological is representative of the status or the value of the physiological property of the subject 104 during the second epoch. The status or the value of the physiological property may be present only during the second epoch, or may extend beyond the first epoch to one or more adjacent epochs.

FIG. 4 depicts a plurality of probability distributions according to the first embodiment. In FIG. 4, the probability distributions of 10 epochs are depicted. The total probability of each probability distribution is set at the value 1. The sleep stage classes that are used are the Wake, N1, N2, N3 and REM. Wake is the sleep stage class in which the subject 104 is awake. N1 and N2 are light sleep stage classes. N3 is a deep sleep stage class. REM is the sleep stage class in which the subject 104 has Rapid Eye Movements.

In case a probability of one of the sleep stage classes is close to 0, there is a low probability that the subject 104 has a sleep stage in that sleep stage class during that epoch. In case a probability of one of the sleep stage classes is close to 1, there is a high probability that the subject 104 has a sleep stage in that sleep stage classes during that epoch.

In epoch 1, the probability distribution shows only the Wake, and none of the other sleep stage classes. It is highly probable that the subject 104 is awake during epoch 1. In epochs 2 and 3, Wake is reducing compared to epoch 1, while N1 is becoming the largest probability. It is likely that the subject 104 is in light sleep during epoch 3. During epochs 4-6, N3 is becoming the largest probability. Epochs 1-6 show a natural progression of the subject 104 from being awake via light sleep to deep sleep. There are differences between the probability distributions of epochs 1-6. The continuity metric regarding these differences represents that the differences are small and consistent with natural sleep without an arousal present.

However, there is a large difference between the probability distributions of epochs 6 and 7. In epoch 6, the probability of N3 is about 0.9, whereas the probability of N3 is about 0.1 in epoch 7. Further, epoch 7 shows a large probability of 0.8 of N1 and N2. This large difference indicates an abrupt change from deep sleep N3 to light sleep N1 and N2. The continuity metric regarding this large difference represents the presence of an arousal.

From epoch 7-10, N1 gradually decreases, whereas N3 and REM gradually increase. The continuity metric regarding epochs 7-10 represents that there are small differences which are consistent with natural sleep without an arousal present.

So returning to the computer-implemented method depicted in FIG. 3, the first epoch is, for example, epoch 6, and the second epoch is epoch 7.

The first epoch and the second epoch are adjacent to each other. The first epoch and the second epoch are adjacent to each other in time.

The first probability distribution is representative of a classification of the first epoch as one of the sleep stage classes. The second probability distribution is representative of a classification of the second epoch as another one of the sleep stage classes. Based on the largest probability, epoch 6 is classified as N3, whereas epoch 7 is classified as either N1 or N2.

Optionally, the computer-implemented method makes use of the multiple probabilities in a probability distribution. The probability distribution comprises multiple probabilities, one for each of the sleep stage classes. For example, the probabilities for epoch 6 are 0.05 for N1, 0.05 for N2 and 0.9 for N3. For example, the probabilities for epoch 7 are 0.4 for N1, 0.4 for N2, 0.1 for N3 and 0.1 for REM. The continuity metric is based on a difference between probabilities of corresponding sleep stage classes in the first epoch and the second epoch. For example, the continuity metric is based on the difference for N1 of 0.4-0.05=0.35. For example, the continuity metric is based on the difference for N2 of 0.4-0.05=0.35. For example, the continuity metric is based on the difference for N3 of 0.1-0.9=-0.8. For example, the continuity metric is based on the difference for REM of 0.1-0.0=0.1.

Optionally, the computer-implemented method comprises receiving at least one additional signal comprising information about at least one additional probability distribution of at least one additional epoch. The first epoch, the second epoch and the at least one additional epoch are within a timeframe of an arousal. The computer implemented method comprises determining the continuity metric based on a difference between the first probability distribution, the second probability distribution, and the at least one additional probability distribution. The timeframe of the arousal may extend over more epochs than only the first epoch and the second epoch, e.g., epochs 6 and 7. Therefore, 1 or 2 or more additional epochs are optionally included in the computer-implemented method to determine the continuity metric. For example, the continuity metric is based on the additional probability distribution of epoch 5, or of epoch 8, or of epoch 4 or of epoch 9. For example, the continuity metric is based on the additional probability distributions of epoch 4 and epoch 5. For example, the continuity metric is based on the additional probability distributions of epoch 8 and epoch 9. For example, the continuity metric is based on the additional probability distributions of epoch 5 and epoch 8.

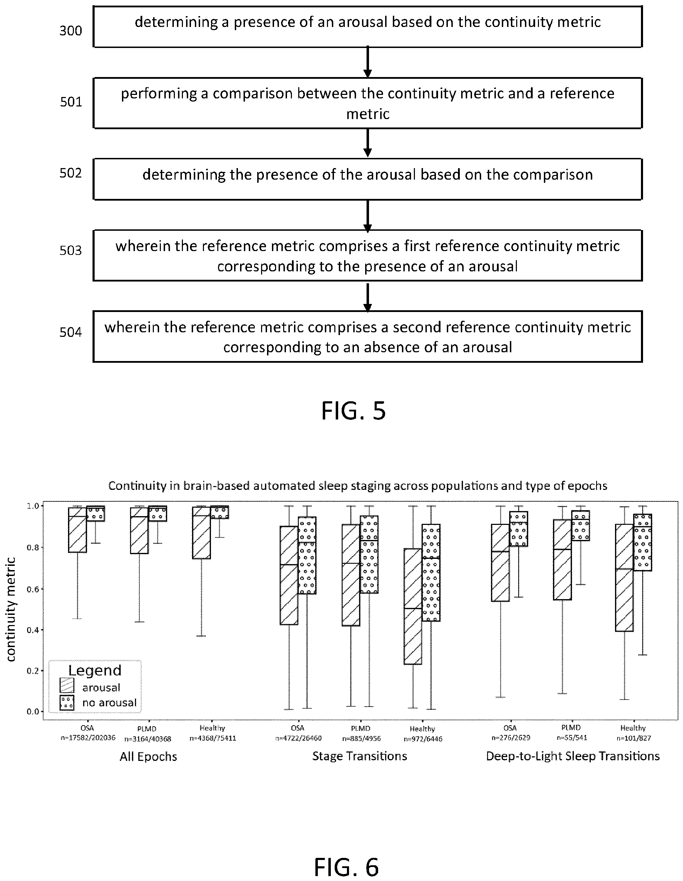

FIG. 5 depicts a computer-implemented method according to a second embodiment of the invention. The second embodiment has, for example, the same features as the first embodiment. For example, the computer-implemented method according to the second embodiment is performed by the arousal detection system 106. In the second embodiment, the computer-implemented method comprises, in 300, determining the presence of the arousal. Determining the presence of the arousal comprises, in 501, performing a comparison between the continuity metric and a reference metric. The computer-implemented method comprises, in 502, determining the presence of the arousal based on the comparison.

As an option in the second embodiment, the reference metric comprises, in 503, a first reference continuity metric corresponding to the presence of an arousal. As an option in the second embodiment, the reference metric comprises, in 504, a second reference continuity metric corresponding to an absence of an arousal.

As an option in the first embodiment or the second embodiment, the computer-implemented method defines defining the continuity metric with the formula:

$$\text{Continuity metric} = 1 - \frac{1}{2}\sum_i |p(x_i(t)) - p(x_i(t+1))| \qquad (1)$$

wherein $p(x_i(t))$ resembles a probability for each sleep stage in the first epoch,
wherein $p(x_i(t+1))$ resembles a probability for each sleep stage in the second epoch.

Formula (1) calculates the sum of the absolute differences in the probabilities between two epochs. By multiplying the sum by 1/2 and subtracted from 1, the continuity metric was normalized to a value between 0 and 1, where 0 represents no continuity and 1 represents maximum continuity.

The following relates to a study performed using the invention. A brain-based automated sleep stage classification model, e.g., Somnolyzer, and a heart rate variability (HRV)-based automated sleep stage classification model, e.g., CReSS, were used to classify sleep stages into four sleep stage classes. The brain-based automated sleep stage classification model is configured to classify the sleep stages based on information about brain activity of the subject 104. The HRV-based automated sleep stage classification model is configured to classify the sleep stages based on cardiac information of the subject 104. The four sleep stage classes are wake, light sleep, deep sleep, and REM sleep. The continuity metric was calculated according to (1). The length of an epoch is 30 seconds.

FIG. 6 depicts the continuity metric based on the probability distributions generated by the brain-based automated sleep stage classification model. FIG. 6 depicts three groups of boxplots. Each group has three pairs of boxplots. In each group, the left pair of boxplots represents the continuity metric for subjects with obstructive sleep apnea, OSA. In each group, the middle pair of boxplots represents the continuity metric for subjects with periodic limb movement disorder, PLMD. In each group, the right pair of boxplots represents the continuity metric for healthy subjects. The healthy subjects do not have OSA or PLMD. Further, FIG. 6 depicts with the 'n' below the x-axis the number of epochs, in which either an arousal was present or not, that were taken into account to create the boxplots. The boxplots were created based on sleep sessions of multiple subjects.

In the group on the left, the continuity metric is depicted for all epochs that were measured during the sleep sessions. These epochs include adjacent epochs that are classified as the same sleep stage class, and adjacent epochs that have different sleep stage classes. The boxplots in the group on the left indicate that the continuity metric is close to 1. The boxplots in the group on the left indicate that the continuity metric is substantially lower than 1 in case an arousal is present. Whether a subject 104 has OSA or PLMD, or whether the subject 104 is healthy does not significantly affect the boxplots.

In the group in the middle, the continuity metric is depicted for all epochs that represent a sleep stage transition. The continuity metric is based on two epochs, wherein one of the two epochs is classified to a different sleep stage class than the other of the two epochs. For example, the continuity metric is based on at least two epochs, wherein one of the two epochs is classified to a different sleep stage class than one of the other epochs. For the three groups of subjects, the continuity metric is substantially higher in case there is no arousal compared to in case there is an arousal. The difference between the continuity metric in case of an arousal or in case of no arousal is the largest for healthy subjects. However, also for subjects with OSA or PLMD, there is a substantial difference between the continuity metric in case of an arousal and in case of no arousal.

In the group on the right, the continuity metric is depicted for all epochs that represent a sleep stage transition from the deep sleep class to the light sleep class. The continuity metric is based on two epochs, wherein the first epoch is classified as the deep sleep class, and wherein the second epoch is classified as the light sleep class. For the three groups of subjects, the continuity metric is substantially higher in case there is no arousal compared to in case there is an arousal. The difference between the continuity metric in case of an arousal and in case of no arousal is the largest for healthy subjects. However, also for subjects with OSA or PLMD, there is a substantial difference between the continuity metric in case of an arousal and in case of no arousal.

Based on the information from FIG. 6 it is depicted that the continuity metric representing the continuity between sleep stages provides a base to determine the presence of an arousal. Further, the information from FIG. 6 is optionally used as a reference metric. For example, a value from one or more boxplots is used as a reference metric to compare a continuity metric of a sleep session of a subject 104. For example, the value is a mean, or median, or a maximum, or a minimum value of one of the boxplots. Depending on whether the subject 104 has OSA, PLMD, or neither OSA nor PLMD, a specific boxplot is, for example, selected for use in the reference metric. For example, the reference metric comprises information from a boxplot representing the presence of an arousal, and from a boxplot representing an absence of an arousal.

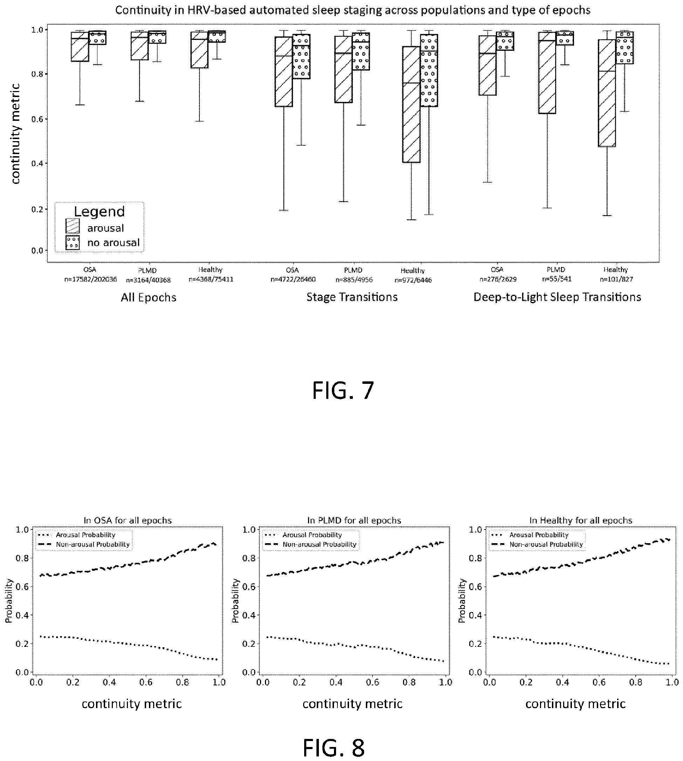

FIG. 7 depicts the continuity metric based on the probability distributions generated by the HRV-based automated sleep stage classification model. Similar to FIG. 6, FIG. 7 depicts three groups of boxplots. Each group has three pairs of boxplots. In each group, the left pair of boxplots represents the continuity metric for subjects with obstructive sleep apnea, OSA. In each group, the middle pair of boxplots represents the continuity metric for subjects with periodic limb movement disorder, PLMD. In each group, the right pair of boxplots represents the continuity metric for healthy subjects. The healthy subjects do not have OSA or PLMD. Further, FIG. 7 depicts with the 'n' below the x-axis the number of epochs, in which either an arousal was present or not, that were taken into account to create the boxplots. The boxplots were created based on sleep sessions of multiple subjects.

In the group on the left, the continuity metric is depicted for all epochs that were measured during the sleep sessions. These epochs include adjacent epochs that are classified as the same sleep stage class, and adjacent epochs that have different sleep stage classes. The boxplots in the group on the left indicate that the continuity metric is close to 1. The boxplots in the group on the left indicate that the continuity metric is substantially lower than 1 in case an arousal is present. Whether a subject 104 has OSA or PLMD, or whether the subject 104 is healthy does not significantly affect the boxplots.

In the group in the middle, the continuity metric is depicted for all epochs that represent a sleep stage transition. The continuity metric is based on two epochs, wherein one of the two epochs is classified to a different sleep stage class than the other of the two epochs. For example, the continuity metric is based on at least two epochs, wherein one of the two epochs is classified to a different sleep stage class than one of the other epochs. For the three groups of subjects, the continuity metric is substantially higher in case there is no arousal compared to in case there is an arousal. The difference between the continuity metric in case of an arousal and in case of no arousal is the largest for healthy subjects. However, also for subjects with OSA or PLMD, there is a substantial difference between the continuity metric in case of an arousal and in case of no arousal.

In the group on the right, the continuity metric is depicted for all epochs that represent a sleep stage transition from the deep sleep class to the light sleep class. The continuity metric is based on two epochs, wherein the first epoch is classified as the deep sleep class, and wherein the second epoch is classified as the light sleep class. For the three groups of subjects, the continuity metric is substantially higher in case there is no arousal compared to in case there is an arousal. The difference between the continuity metric in case of an arousal and in case of no arousal is the largest for healthy subjects. However, also for subjects with OSA or PLMD, there is a substantial difference between the continuity metric in case of an arousal and in case of no arousal.

Based on the information from FIG. 7, it is depicted that the continuity metric representing the continuity between sleep stages provides a base to determine the presence of an arousal. Further, the information from FIG. 7 is optionally used as a reference metric. For example, a value from one or more boxplots is used as a reference metric to compare a continuity metric of a sleep session of a subject 104. For example, the value is a mean, or median, or a maximum, or a minimum value of one of the boxplots. Depending on whether the subject 104 has OSA, PLMD, or neither OSA nor PLMD, a specific boxplot is, for example, selected for use in the reference metric. For example, the reference metric comprises information from a boxplot representing the presence of an arousal, and from a boxplot representing an absence of an arousal.

FIGs 8-10 depict a probability of an arousal based on the continuity metric. Based on the boxplots of FIGs 6 and 7, it is shown that the presence or absence of an arousal can be estimated based on the continuity metric. Based on the continuity metric, a probability of an arousal, and a probability of no arousal are depicted in FIGs 8-10. Based on a plurality of continuity metrics expanding a substantial part or all of a sleep session of the subject 104, the number of arousals can be estimated. The number of arousals provides an important clinical insight into the health and the quality of sleep of the subject 104.

Each of FIGs 8-10 has three graphs. Each graph depicts the probability of an arousal or of no arousal on the y-axis, and the continuity metric on the x-axis. The left graph depicts the probability for subjects with OSA. The middle graph depicts the probability for subjects with PLMD. The right graph depicts the probability for healthy subjects. Healthy subjects do not have OSA or PLMD.

FIG. 8 depicts the probability for an arousal and for no arousal for all epochs, so including sleep stage transition epochs and epochs without sleep stage transition. For example, on average, for subjects having OSA, 8% of the epochs contain an arousal. The left graph of FIG 8 depicts that there is a >25% chance for subjects with OSA that there is an arousal when the continuity is below 0.2, while the there is only a 7% chance there is an arousal when continuity is above 0.9. Similar values are shown in the middle graph for subjects with PLMD, and in the right graph for healthy subjects. Based on the information of FIG. 8, the number of arousals during a sleep session can be estimated by taking the probability for an arousal and for no arousal into account for each continuity metric.

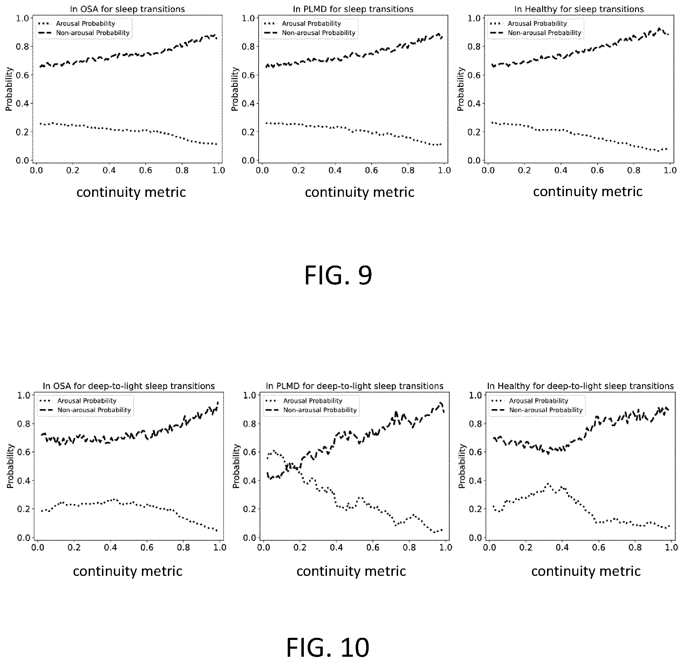

FIG. 9 depicts the probability for an arousal and for no arousal for epochs that show a sleep stage transition. Based on the information of FIG. 9, the number of arousals during a sleep session can be estimated by taking the probability for an arousal and for no arousal into account for each continuity metric.

FIG. 10 depicts the probability for an arousal and for no arousal for epochs that show a sleep stage transition from the deep sleep stage to the light sleep stage. Based on the information of FIG. 10, the number of arousals during a sleep session can be estimated by taking the probability for an arousal and for no arousal into account for each continuity metric. For example, for subjects with PLMD, 9% of the deep-to-light-sleep transitions contain an arousal. However, when there is a sleep stage transition from the deep sleep stage to the light sleep stage with a continuity metric above 0.9, there is a below 5% chance the sleep stage transition is accompanied by an arousal. When continuity metric is below 0.3, there is a 40% chance the sleep stage transition is accompanied by an arousal. Based on FIG. 10, the number of arousals during sleep stage transitions from the deep sleep stage to the light sleep stage can be estimated based on the 8 times higher chance in low continuity.

The skilled person would be readily capable of developing the processing system 200 for carrying out any herein described method. Thus, each step of the flow charts may represent a different action performed by the processing system 200, and may be performed by a respective module of the processing system 200.

The processing system 200 can be implemented in numerous ways, with software and/or hardware, to perform the various functions required. The processing system 200 typically employs one or more microprocessors that may be programmed using software (e.g., microcode) to perform the required functions. The processing system 200 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system 200 may be embodied as a digital and/or analog processing system 200.

In various implementations, the memory 204 may comprise one or more storage media such as volatile and non-volatile computer memory such as RAM, PROM, EPROM, and EEPROM. The storage media may be encoded with one or more programs that, when executed on one or more processing system 200s and/or controllers, perform the required functions. Various storage media may be fixed within a processing system 200 or controller may be transportable, such that the one or more programs stored thereon can be loaded into the processing system 200.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A computer-implemented method for detecting an arousal in a sleep session of a subject (104), the computer-implemented method comprising:

   receiving (300) a first signal comprising information about a first probability distribution of sleep stage classes during the first epoch;
   receiving (301) a second signal comprising information about a second probability distribution of the sleep stage classes during the second epoch;
   determining (302) a continuity metric representative of a difference between the first probability distribution and the second probability distribution;
   determining (303) a presence of an arousal based on the continuity metric.

2. The computer-implemented method according to claim 1,
   wherein the first epoch and the second epoch are adjacent to each other.

3. The computer-implemented method according to any one of the preceding claims,

   wherein the first probability distribution is representative of a classification of the first epoch as one of the sleep stage classes,
   wherein the second probability distribution is representative of a classification of the second epoch as another one of the sleep stage classes.

4. The computer-implemented method according to any one of the preceding claims, wherein determining the presence of the arousal comprises:

   performing (501) a comparison between the continuity metric and a reference metric; and
   determining (502) the presence of the arousal based on the comparison.

5. The computer-implemented method according to claim 4, wherein the reference metric comprises a first reference continuity metric corresponding to the presence of an arousal.

6. The computer-implemented method according to claim 4 or 5,
   wherein the reference metric comprises a second reference continuity metric corresponding to an absence of an arousal.

7. The computer-implemented method according to any one of the preceding claims,

   wherein the probability distribution comprises multiple probabilities, one for each of the sleep stage classes,
   wherein the continuity metric is based on a difference between probabilities of corresponding sleep stage classes in the first epoch and the second epoch.

8. The computer-implemented method according any one of the preceding claims, comprising:

   receiving at least one additional signal comprising information about at least one additional probability distribution of at least one additional epoch;
   wherein the first epoch, the second epoch and the at least one additional epoch are within a timeframe of an arousal;
   determining the continuity metric based on a difference between the first probability distribution, the second probability distribution, and the at least one additional probability distribution.

9. The computer-implemented method according to any one of the preceding claims, defining the continuity metric with the formula:

$$\text{Continuity metric} = 1 - \frac{1}{2}\sum_i |p(x_i(t)) - p(x_i(t+1))|$$

wherein $p(x_i(t))$ resembles a probability for each sleep stage in the first epoch,
wherein $p(x_i(t+1))$ resembles a probability for each sleep stage in the second epoch.

10. The computer-implemented method according to any one of the preceding claims, comprising:

receiving a first physiological signal (131-134) representative of a physiological property of the subject (104) during the first epoch;
receiving a second physiological signal representative (131-134) of the physiological property of the subject (104) during the second epoch,
wherein the physiological property comprises information about a sleep stage of the subject (104);
generating the first probability distribution based on the first physiological signal (131-134);
generating the second probability distribution based on the second physiological signal (131-134).

11. The computer-implemented method according to claim 10,
comprising using an automated sleep stage classification model (208) for:

generating the first probability distribution based on the first physiological signal (131-134);
generating the second probability distribution based on the second physiological signal (131-134);
classifying the first epoch as one of the sleep stage classes; and
classifying the second epoch as one of the sleep stage classes.

12. The computer-implemented method according to claim 10 or 11, wherein the physiological property is related to at least one of a brain activity, a cardiac property, and a respiratory property of the subject (104) during the sleep session.

13. A computer program product, comprising instructions which, when executed by a processing system (200), cause the processing system (200) to carry out the computer-implemented method of any one of claims 1-12.

14. An arousal detection system (106) for detecting an arousal in a sleep session of a subject (104), the arousal detection system (106) comprising:

a processing system (200) configured to perform the computer-implemented method of any one of claims 1-12;
a sensor interface (202) coupled to the processing system (200),
wherein the sensor interface (202) is configured to be coupled to at least one sensor (121-124) for generating the physiological signal (131-134) representative of the physiological property;
wherein the sensor interface (202) is configured to provide the first physiological signal (131-134) and the second physiological signal (131-134) to the processing system (200); and
an output generator (206) configured to generate an output signal (216) representative of the presence of the arousal in response to the processing system (200) determining the presence of the arousal.

15. A respiratory support device (100) for providing a pressurized airflow (102) to a subject (104), the respiratory support device (100) comprising:

the arousal detection system (106) according to claim 14;
an airflow source (108) adapted to generate the pressurized airflow (102); and
a patient interface (110) adapted to provide the pressurized airflow (102) to the subject (104).

FIG. 1

FIG. 2

| 300 | receiving a first signal comprising information about a first probability distribution of sleep stage classes during the first epoch |
|---|---|

↓

| 301 | receiving a second signal comprising information about a second probability distribution of the sleep stage classes during the second epoch; |
|---|---|

↓

| 302 | determining a continuity metric representative of a difference between the first probability distribution and the second probability distribution; |
|---|---|

↓

| 303 | determining a presence of an arousal based on the continuity metric |
|---|---|

## FIG. 3

FIG. 4

| | |
|---|---|
| 300 | determining a presence of an arousal based on the continuity metric |
| 501 | performing a comparison between the continuity metric and a reference metric |
| 502 | determining the presence of the arousal based on the comparison |
| 503 | wherein the reference metric comprises a first reference continuity metric corresponding to the presence of an arousal |
| 504 | wherein the reference metric comprises a second reference continuity metric corresponding to an absence of an arousal |

## FIG. 5

Continuity in brain-based automated sleep staging across populations and type of epochs

Legend
arousal
no arousal

| All Epochs | | | Stage Transitions | | | Deep-to-Light Sleep Transitions | | |
|---|---|---|---|---|---|---|---|---|
| OSA n=17582/202036 | PLMD n=3164/40368 | Healthy n=4368/75411 | OSA n=4722/26460 | PLMD n=885/4956 | Healthy n=972/6446 | OSA n=276/2629 | PLMD n=55/541 | Healthy n=101/827 |

## FIG. 6

FIG. 7

FIG. 8

FIG. 9

FIG. 10

Europäisches
Patentamt

European
Patent Office

Office européen
des brevets

# EUROPEAN SEARCH REPORT

**Application Number**

EP 24 17 0984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2013/319415 A1 (MULCAHY DAVID [AU] ET AL) 5 December 2013 (2013-12-05) * paragraphs [0050] - [0055], [0098] - [0102]; claims 97-101 * | 1-15 | INV. A61B5/00 |
| Y | WO 2018/070935 A1 (NAT UNIV SINGAPORE [SG]) 19 April 2018 (2018-04-19) * paragraphs [0027] - [0032], [0038] * | 1-15 | |
| Y | MPORAS IOSIF ET AL: "Improving sleep stage classification from electroencephalographic signals by fusion of contextual information", 2015 IEEE 15TH INTERNATIONAL CONFERENCE ON BIOINFORMATICS AND BIOENGINEERING (BIBE), IEEE, 2 November 2015 (2015-11-02), pages 1-4, XP032839544, DOI: 10.1109/BIBE.2015.7367736 [retrieved on 2015-12-28] * II. SLEEP STAGE CLASSIFICATION USING CONTEXTUAL INFORMATION, p. 2; figure 1 * | 1-15 | |
| A | US 2015/190086 A1 (CHAN ALEXANDER [US] ET AL) 9 July 2015 (2015-07-09) * paragraphs [0024], [0035] - [0044] * | 1-15 | TECHNICAL FIELDS SEARCHED (IPC) A61B G16H A61M |

The present search report has been drawn up for all claims

| Place of search | Date of completion of the search | Examiner |
|---|---|---|
| Berlin | 1 October 2024 | Trachterna, Morten |

EPO FORM 1503 03.82 (P04C01)

## ANNEX TO THE EUROPEAN SEARCH REPORT
## ON EUROPEAN PATENT APPLICATION NO.

EP 24 17 0984

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2024

| Patent document cited in search report | Publication date | Patent family member(s) | Publication date |
|---|---|---|---|
| US 2013319415 A1 | 05-12-2013 | US 2009038616 A1 | 12-02-2009 |
| | | US 2013319415 A1 | 05-12-2013 |
| | | US 2016346492 A1 | 01-12-2016 |
| | | US 2020094004 A1 | 26-03-2020 |
| | | US 2024024598 A1 | 25-01-2024 |
| WO 2018070935 A1 | 19-04-2018 | SG 10201608507P A | 30-05-2018 |
| | | WO 2018070935 A1 | 19-04-2018 |
| US 2015190086 A1 | 09-07-2015 | US 2015190086 A1 | 09-07-2015 |
| | | WO 2015103558 A1 | 09-07-2015 |

EPO FORM P0459

For more details about this annex : see Official Journal of the European Patent Office, No. 12/82